# EUROPEAN PATENT APPLICATION

(11) **EP 4 249 584 A1**
(43) Date of publication of application: **27.09.2023**
(21) Application number: 22163492.6
(22) Date of filing: 22.03.2022
(51) Int. Cl.: C12N 5/00

(54) **A POLYMER ASSEMBLY FOR GROWING CELLS**

(71) Applicant: CATALYA, 4020 Liège (BE)
(72) Inventor: GRANDFILS, Christian, 4020 Liège (BE); SEVRIN, Chantal, 4020 Liège (BE)
(74) Representative: Calysta NV

(57) **Abstract**

A coated surface with a polymer substantially consisting of a succession of monomers according to the formula I and its use for growing cells.

## Description

### Technical Field

The present invention relates to a polymer for use in the coating of surfaces, useful for growing cells.

### Prior art

Animal cell cultures are daily used in routine either for various *in vitro* applications amongst other for R & D activities, diagnostic applications, cytotoxicity and pharmacological assays. Animals cells are also broadly used in the pharmaceutical industries in order to produce biopharmaceutical active ingredients, such as recombinant proteins, vaccines, nucleic acids (DNA, mRNA,..), exosomes, viral vectors. Today animal cells are also conceivable as an end-therapeutic product in the frame of cell therapy and tissue engineering for organ repair or even organ replacement.

Most of these animal cells are so-called adherent animal cells, because, when cultured *in vitro,* their short-term survival is strictly dependent on their ability to adhere quickly to a solid surface, which has to be tailored to promote cell attachment, then cell proliferation, followed afterwards by cells differentiation. During these steps, cells undergo morphological changes arising from their passive deformation, but also the reorganization of their cytoskeleton. *In vivo,* but also *in vitro* this cell adhesion process is essential for cell communication and regulation and is crucial for cell development and maintenance.

This dependence of the survival of animal cells to their adhesion to a solid surface is imposing to develop enough surface accessible to the cells when trying to amplify them to large-scale level. Indeed, grossly taking into account that the typical mean surface area required per cell is close to 350 µm², a total area of 0.2 m² is required to produce a therapeutic dose of stem cells for cell therapy (i.e. 500 million of cells) and that a total area of 2000 m² is typically needed to cultivate animal cells in a 2000 L bioreactor for vaccine production.

At the laboratory level, cells are cultivated on 2D surfaces using typically multiwell plates or T-Flasks whilst the biopharmaceutical industry is adopting 3D cell culture support and doing cell cultivation in bioreactors. Both 2D or 3D surface material needs to be optimized in order to promote cell adhesion and proliferation.

*In vivo,* cell adhesion to either other cells or to the extracellular matrix is mediated by proteins, such as integrins. These transmembrane proteins act as transducer to the cell cytoskeleton extensors and actin filaments promoting the formation of focal adhesion complex.

In the case of *in vitro* cell culture, no specific biological ligands are applied on the surface of the materials used. Cell adhesion mostly depends of lesser specific interactions & weak bonds, which can rely on hydrophobic or van der Waals interactions. However, animal cells possess a negative surface-charge at physiological pH. Interestingly, cells can however be cultivated on both positive (DEAE-ion exchanger) or negatively charged surfaces. Interestingly, the experience has demonstrated that it is not the polarity of the charges, but the surface charge density on the culture surface which is key to the cell adhesion. Critical values need to be achieved to promote cell adhesion and proliferation. This optimal value corresponds to a discrete range of surface charges density which is for negatively charged surfaces within the range of 2 - 10 charges /nm² and about 600 charges/nm² for positively charged surfaces. Above these values, the cell growth is inhibited or cells are dying, and below those values, cell adhesion is very poor. This critical surface charge density is also affected by the interaction between proteins or polymers contained in the culture medium and which are adsorbed on the surface, and thus exposed to the cells.

The material most widely used for *in vitro* cell culture for 2D animal cell culture is polystyrene. This polymer is very hydrophobic and devoid from any charges. Without any treatment, polystyrene does not promote animal cell adhesion and is therefore only suitable for the culture of non-adherent cells. In contrast, when polystyrene surfaces are chemically or physically treated to introduce ionic groups, typically anionic groups present on tissue culture polystyrene (TCPS), its surface is rapidly covered by a thin layer of proteins upon incubation with serum-containing culture medium, allowing the deposition of extracellular matrix, which favour cell adhesion.

If TCPS can be used for several animal cell lines, different strategies have been reported to improve the adhesion of fibroblasts and osteoblasts in *vitro* and to better control this. Either functional non-specific groups have been anchored on the surface of the polystyrene in order to increase at least their hydrophilicity and their surface charge density, either ligands recognizing specific cell integrins have been also chemically attached to the material surface. Different synthetic or natural macromolecules such as poly-L-lysine or proteins characteristics of the extracellular matrix, *i.e.* fibronectin, vitronectin, collagen and laminin, have been also either physically adsorbed or chemically grafted on polymer surface.

However, when reaching cell confluence *in vitro,* cells have to be detached from the solid surface in order to ensure a good viability ration. This harvesting process, which should be repeated on a regular basis, every 7 to 10 days, sometimes more frequently, is called a "cell passage". Until now this cell detachment, both at laboratory scale and the industrial scale, request to use proteolytic enzymes to hydrolyse the integrins or part of their extra-cellular fragments allowing to release eukaryotic cells from the surface. This harvesting process is suffering from several drawbacks, chief amongst which are the cost of the enzyme treatment and the risk of cell apoptosis by anoikis. Therefore, this enzymatic treatment should be kept to a minimum impact, with a rapid neutralization of the proteolytic enzymes by addition of inhibiting proteins. For in *vivo* applications, the enzymatic contamination of stem cells can raise safety concerns. This enzymatic treatment, alone or combined with calcium chelating agents, such as EDTA, has been also reported as poorly efficient, especially when dealing with stem cell detachment from various microcarriers.

Alternative strategies have been evaluated to replace enzymatic treatment by alternative methodologies which could rely on simple physico-chemical triggering events more compatible with eucaryotic cell viability. Different stimuli could be used in order to change the physico-chemical behaviour of polymers coated to a surface and potentially to control cell adhesion more easily. Amongst several techniques described, one can mention: change in temperature, application of an electric field, local dissipation of energy under the form of light, ultrasound, magnetic fields or the application of mechanical agitations.

The thermal-inducible polymers, N-Isopropylacrylamide (pNIPAM) is the most studied thermal-inducible polymers in its category, with a phase transition around 32°C, i.e. close to the physiological conditions. Above the LCST (Lower Critical Solution Temperature) of 32°C the hydrogen bonds responsible of the solvation of pNIPAM are essentially broken, inducing a change of its conformation to a collapsed globular and hydrophobic form favourable to cell adhesion. Below 32°C, pNIPAM have hydrated, hydrophilic and mobile chains which are ensuring steric repulsion forces inhibiting protein adsorption promoting animal cell detachment. However, in practice, the efficiency and kinetics of this non enzymatic cell detachment process has been disclosed to be low and has not been applied on any commercial cell culture materials today.

The pH is an important environmental parameter for biomedical applications, because pH changes occur in many specific or pathological compartments. The key element for pH responsive polymers is the presence of ionisable, weak acidic or basic moieties that attach to a hydrophobic backbone, such as polyelectrolytes. Upon ionization, the electrostatic repulsions of the generated charges (anions or cations) cause a dramatic extension of coiled chains. The ionization of the pendant acidic or basic groups on polyelectrolytes can be partial, due to the electrostatic effect from other adjacent ionized groups. Accordingly small changes in pH can induce, very abruptly, phase transition in pH responsive polymers. Some well-known pH responsive polymers are chitosan, albumin, gelatine, poly(acrylic acid), poly(methacrylic acid-g-ethylene glycol), poly(ethylene imine) (PEI) or poly(lysine) (PL). However no smart surface able to control cell adhesion and cell detachment have been demonstrated successful until now adopting those list of polymers

Hence, unfortunately, the prior art's methods are not efficient enough.

### Brief summary of the invention

It is the object of the invention to improve the *in vitro* culture of animal cells by enhancing both (i) the cell adhesion process and (ii) the cell detachment, when needed.

According to the invention, this object is solved by providing a simple, rapid and cost-effective physical coating of a cell culture substrate by a polymer (herein after referred to as PDMAEMA) consisting essentially of a succession of monomers according to the formula I: 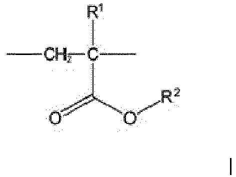
wherein R¹ represents a hydrogen atom or a straight or branched chain alkyl group from 1 to 6 carbon atoms;
wherein R² represents a straight or branched chain alkyl group from 1 to 6 carbon atoms, which is substituted by a protonable amino group.

This poly(N,N-diakylamino ethylmethacrylates) PDMAEMA, when coated on the surface of materials used in cell culture, promotes either cell adhesion or cell detachment through a moderate and quick change in pH of the culture medium. This is due to very important change in the polymer charge density, being mostly positive at pH 7.4 and neutral close to pH 8.0.

A first aspect of this invention is a method to grow animal cells comprise the steps of:
- selecting a surface
- coating the said surface with a polymer substantially consisting of a succession of monomers according to the formula I
- growing these animal cells on this coated surface for a ((pre)determined) period of time so that these animal cells adhere to this coated surface.

A second aspect of this invention is a composition comprising a surface of materials used in cell culture and a coating of a polymer substantially consisting of a succession of monomers according to the formula I.

### Brief description of the Drawings

Figure 1. Microscopic observations of L929 fibroblasts 4 or 7 days after culture on 24 multiwell made of TCPS or PS.
Figure 2. Microscopic observations of L929 fibroblasts 4 days after culture on 24 multiwell made of TCPS or PS without or with coating PDMAEMA solutions of 1 or 10 mg/mL
Figure 3. Microscopic observations of L929 fibroblasts 7 days after culture on 24 multiwell made of TCPS or PS without or with coating PDMAEMA solutions of 1 or 10 mg/mL
Figure 4. Microscopic observations of L929 fibroblasts 1, 5 and 7 days after culture on 24 multiwell made of TCPS without or with coating PDMAEMA solutions of concentration of 0 ; 0.1 ; 0.25 ; 0.75 or 1 mg/mL.
Figure 5. Microscopic observations of L929 fibroblasts cultured for 5 days on 24 multiwell made of TCPS, without or with coating PDMAEMA solutions (1 mg/mL) before and after their harvesting either by trypsinization or incubation at pH 8.0.
Figure 6. Microscopic observations of L929 fibroblasts cultured for 5 days on 24 multiwell made of PS coated with PDMAEMA (1 mg/mL) before and after cell harvesting by incubation in DMEM (Dulbecco's Modified Eagle Medium) at pH 8.0.
Figure 7. Microscopic observations of L929 fibroblasts cultured for 7 days on 24 multiwell made of TCPS, without or with coating PDMAEMA (1 mg/mL) or on PS coated with PDMAEMA (1 mg/mL). These observations have been taken before and after cell harvesting either by trypsinisation or incubation at pH 8.0.
Figure 8. MTT (Cell viability assay which relies on the reduction of the tetrazolium dye MTT, i.e. 3-(4,5-dimethylthiazol-2-yl)-2,5-diphenyltetrazolium bromide) counting of fibroblasts L929 after culture for 7 days on 24 multiwell made of eitherTCPS or PS, without or with coating PDMAEMA solutions (1 mg/mL). Before cell counting the cells have been harvested either by trypsinisation or incubation at pH 8.0.
Figure 9. Microscopic observations of L929 fibroblasts cultured for 5 days on 24 multiwell made of TCPS or TCPS coated with PDMAEMA (1 mg/mL) after their harvesting by either trypsinisation or incubation at pH 8.0. The cells have been afterwards seeded on 96 multiwells TCPS for 2h before observing under microscopy and carrying out the MTT.
Figure 10. MTT counting of fibroblasts L929 after culture for 5 or 7 days on 24 multiwell made of either TCPS, without or with coating PDMAEMA solutions (0.1 ; 0.25; 0.75 or 1 mg/mL). Before cell counting the cells have been harvested either by trypsinisation or incubation at pH 8.0.
Figure 11. Microscopic observations of Vero cells after thawing, seeding and cultured for 7 days on 12 multiwell made of TCPS or TCPS coated with PDMAEMA (1 mg/mL).

### Detailed description of the invention

The inventors have formulated a novel approach of surface functionalization of material, relying on the simple, rapid and cost-effective physical coating of poly(N,N-diakylamino ethylmethacrylates), PDMAEMA, on the surface of materials used in cell culture. Through a moderate and quick change in pH of the culture medium, this polymer promotes either cell adhesion or cell detachment, due to its very important change in charge density, being mostly positive at pH 7.4 and neutral close to pH 8.0.

A first aspect of the present invention is thus a method to grow animal cells, preferably mammalian cells, comprising the steps of:
- selecting a surface,
- coating the said surface with a polymer substantially consisting of a succession of monomers according to the formula I 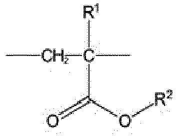 I and
- growing these animal (mammalian and/or stem) cells on this coated surface for a (pre(determined)) period of time so that these animal (mammalian and/or stem) cells adhere to this coated surface,
   wherein R¹ represents a hydrogen atom or a straight or branched chain alkyl group from 1 to 6 carbon atoms;
   wherein R² represents a straight or branched chain alkyl group from 1 to 6 carbon atoms, which is substituted by a protonable amino group;
   wherein this polymer has a mean pKa between 6.0 and less than 7.5 and a charge density at pH 7.0 between 20 and 50% (number of the positively charged monomers:total number of monomers).

This allows the coating of surface(s) with a polymer having a defined density of positive charges.

Preferably, the method of the present invention comprises the preliminary step of determining the optimal charge density of the polymer substantially consisting of a succession of monomers according to the formula I. Advantageously, several surfaces are coated with a diversity of the polymer substantially consisting of a succession of monomers according to the formula I, before application of the culture medium for a predetermined period of time and application of the animal (human and/or stem) cells and the most suitable polymer is selected. Preferably, structures (III) to (XIV) here below are incorporated to a certain extent to the polymer substantially consisting of a succession of monomers according to the formula I, which will fine-tune the charge density.

This allows to identify the ideal polymer, depending (i) on the surface, (ii) on the culture condition and (iii) on the type of cells.

Preferably this animal and/or mammalian and/or stem cells are not human embryonic stem cells.

The surface can be a 2D surface or a 3D surface, which is advantageous for large-scale production.

In the present invention, negatively-charged surfaces to be coated by the polymer of the present invention are preferred. In the context of the present invention, the terminology "negatively charged surfaces" means a surface (an uncoated surface) having a density of negative charges higher than 10/nm², preferably higher than 50/nm², more preferably higher than 100/nm² and/or more negative charges than positive charges per surface unit (uncoated; hence without taking into account the polymers to be adsorbed on the surface, including the polymer of the present invention, or proteins, polycations, polylysine, chitosan..., added through the culture medium or added as a coating).

However, the polymer of the present invention can also be advantageous for neutral or positively charged surfaces, either the surface per se, or a surface further coated by a positively-charged substance (subsequently coated by the polymer according to the present invention).

The method further comprises the advantageous step of detaching the grown animal (mammalian and/or stem) cells upon increasing the pH of the medium to a pH comprised between 7.8 and 8.5 (preferably between 7.9 and 8.2, such as about 8.0; the pH is preferably the highest possible that does not detrimentally affect cell viability). This can be achieved in synergy with the addition of calcium chelators, such as (sterile) EDTA, for instance between 0.5 and 5mM.

The addition of calcium chelators allows a gentle detachment of the cells, without relying on proteases or on treatments that can be toxic for the cells, such as higher pH values.

Preferably, the R¹ is a methyl group.

Preferably, this polymer (used in the method of the present invention or present in the composition of the present invention) is comprising more than 80%, preferably more than 90% and more preferably more than 95% of the monomers according to the formula I, such as between 95% and 99% (number of the monomers according to the formula I:total number of the monomers).

In other words, preferably, in the context of the present invention (method and composition), the wording "substantially consisting of a succession of monomers according to the formula I" means that more than 60%, preferably more than 65%, more than 70%, more than 75% or even more than 80% of the monomers are those according to the formula I (monomers according to the formula I: total number of the monomers, including those forming the alpha and omega extremities), preferably more than 85, 90, 91, 92, 93, 94, 95, 96, 97, 98% are those according to the formula I.

The remaining monomers, herein after referred to as "complementary monomers" are preferably of an acrylate structure with no protonable amine, such as methyl methacrylate, ethyl methacrylate, propyl methacrylate or butyl methacrylate.

This allows to fine-tune the density of the positive charges (at pH between 7.0 and 7.5) of the polymer.

These complementary monomers will thus be incorporated, possibly in the form of a copolymer with the monomer according to the formula (**I**).

Examples of copolymeric (and terpolymeric) structures which could be used include poly[2-(dimethylamino)ethyl methacrylate)-co-acrylic acid] (**III**), poly[2-(dimethylamino)ethyl methacrylate)-co-methacrylic acid] (**IV**), poly[2-(dimethylamino) ethyl methacrylate)-co- poly(ethylene glycol) a- methyl ether, co-acrylate] (**V**), poly[2-(dimethylamino) ethyl methacrylate)-co-poly (ethylene glycol) a- methyl ether, co-methacrylate] (**VI**), poly[2-(dimethylamino) ethyl methacrylate)-co- poly(ethylene glycol)] (**VII**), poly[2-(dimethylamino) ethyl methacrylate)-co- poly(ethylene glycol)] (**VIII**), poly[methacrylic acid-co-2-(dimethylamino) ethyl methacrylate)-co- poly(ethylene glycol) a-methyl ether, co-methacrylate] (**IX**), poly[methyl methacrylate-co-2-(dimethylamino) ethyl methacrylate)-co- poly(ethylene glycol) a-methyl ether, ω-methacrylate] (**X**), poly[trimethylamino) ethyl methacrylate -co-2-(dimethylamino) ethyl methacrylate)-co- poly(ethylene glycol) a-methyl ether, co-methacrylate] (**XI**), poly[trimethylamino) ethyl methacrylate -co-2-(dimethylamino) ethyl methacrylate] (**XII**), poly[2-(dimethylamino) ethyl methacrylate-co-methyl methacrylate-co-butyl methacrylate] of formula (**XIII**); and poly[2-(trimethylamine) ethyl methacrylate chloride-co-ethyl acrylate-co-methyl methacrylate] of formula (**XIV**).

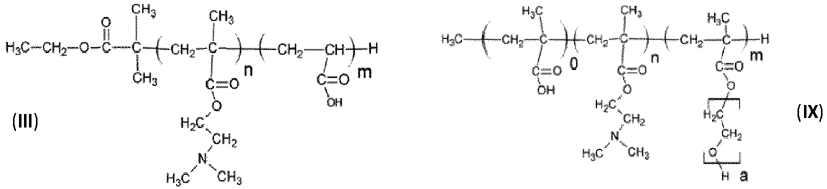

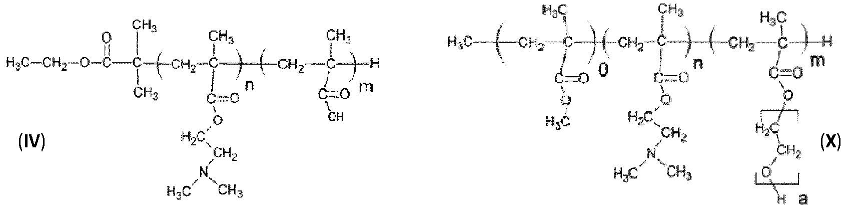

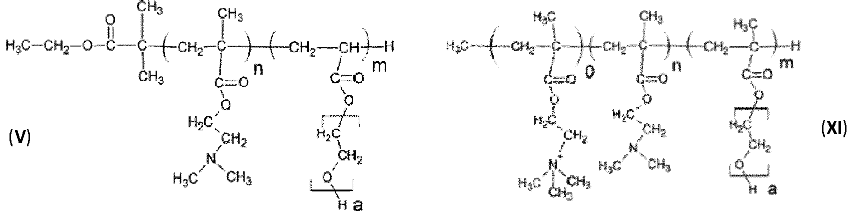

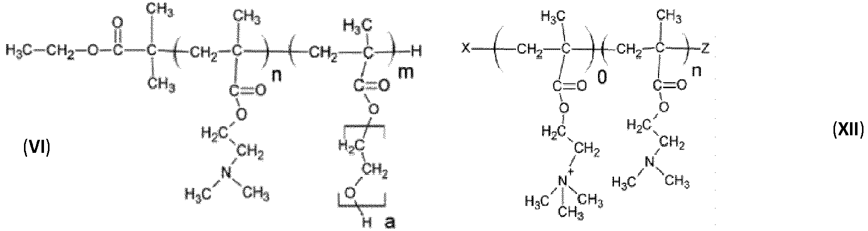

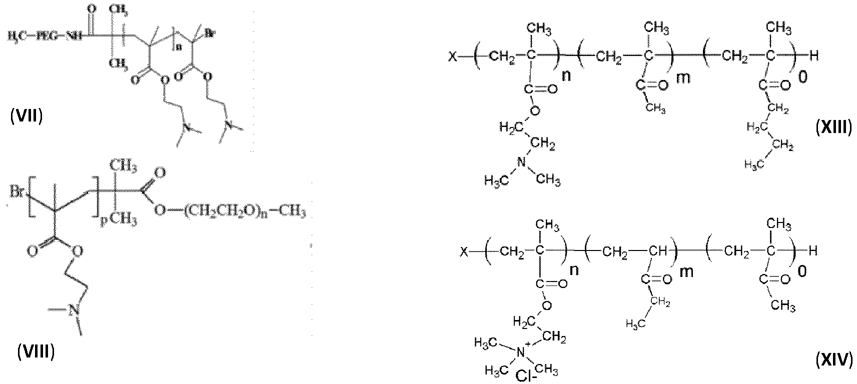

The polymer organization can be adapted depending on the molecule to load: either by random polymerization, or by designing segments consisting of a succession of the monomers according to the Formula I and a succession of the complementary monomers. This allows, for instance, to generate more hydrophobic regions (segments) and more hydrophilic regions (segments) within the same polymer molecule.

The complementary monomers can, possibly, be branched by other oligomeric segments, provided that the polymer keeps its advantageous properties.

The nature, abundance and method of incorporation of the complementary monomers is chosen according to physico-chemical properties needed.

The selection and architecture of the complementary monomers is, of course, more important when they are incorporated in higher amounts, such as when more than 20 %, more than 25% more than 30% or even close to 40% of the monomers are complementary monomers: a proportion of at least 60% of the monomers corresponding to the formula I confers the basic structure and physico-chemical properties, and the complementary monomers fine-tune these properties.

The polymerization process can be performed by several methods. Preferably anionic polymerization or free-radical polymerization are used.

Convenient synthesis protocols are indeed based on free-radical polymerization.

ATRP (Atom Transfer Radical Polymerization) is a preferred method of synthesis, especially when there is no need of a specific architecture of the complementary monomers.

RAFT (Reversible Addition Fragmentation Chain Transfer) polymerization has also been successfully found useful for a better control of the polymer organization.

Preferably, in the method of the present invention, the polymer harbors a charge density at pH 7,0 between 25 and 40%, preferably between 30 and 35% (number of the positively charged monomers:total number of monomers). Conversely, preferably, the polymer of the present invention harbors less than 10% of negative charge density at pH 7,0, preferably less than 5%, more preferably less than 2% (number of the negatively charged monomers:total number of monomers).

Preferably, in the method of the present invention, the polymer is coated on the surface upon incubation at a concentration between 0.1 mg/ml and 1.0 mg/ml, preferably 0.15 mg/ml and 0.5 mg/ml, more preferably between 0.2 mg/ml and 0.3 mg/ml.

Preferably, the polymer is according to the formula II 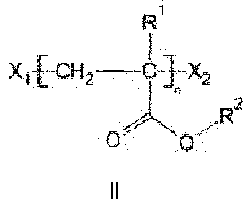
wherein X₁ and X₂ represent the alpha and omega end groups of the said polymer;
wherein the said X₁ and X₂ independently represent a hydrogen atom, a hydroxyl group, an ethyl isobutyrate group, an alkyl group, a halogen group, a carboxylic acid group, an amino group, a methoxy group or an ethoxy group and wherein n is the number of the repetitive monomer units according to the formula I.

A related aspect of the present invention is a composition comprising a surface coated with a polymer substantially consisting of a succession of monomers according to the formula I 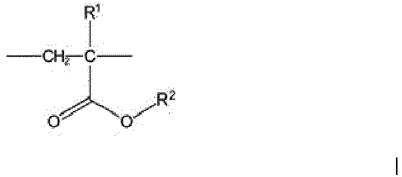
wherein R¹ represents a hydrogen atom or a straight or branched chain alkyl group from 1 to 6 carbon atoms;
wherein R² represents a straight or branched chain alkyl group from 1 to 6 carbon atoms, which is substituted by a protonable amino group.

Preferably, this said polymer has a mean pKa between 6.0 and 7.5 and/or a charge density at pH 7.0 between 20 and 50% (number of the positively charged monomers:total number of monomers).

This allows to adapt in view of the nature of the surface to coat and/or the cell to adhere.

The surface (before coating) is preferably negatively charged, preferably selected from the group of plasma-treated polystyrene, biopolymer, glass.

Preferably the polymer is comprising more than 80%, preferably more than 90% and more preferably more than 95% of the monomers according to the formula I, such as between 95% and 99% (number of the monomers according to the formula I:total number of the monomers), and/or wherein R1 is a methyl group.

Preferably, the polymer harbors a charge density at pH 7,0 between 25 and 40%, preferably between 30 and 35% (number of the positively charged monomers:total number of monomers), and/or harbors less than 10% of negative charge density at pH 7,0, preferably less than 5%, more preferably less than 2% (number of the negatively charged monomers:total number of monomers).

Advantageously, the polymer has been coated on the surface upon incubation at a concentration between 0.01 mg/ml and 1.0 mg/ml, preferably 0.05 mg/ml and 0.5 mg/ml, more preferably between 0.1 mg/ml and 0.3 mg/ml.

Preferably, this composition is further comprising animal cells, preferably mammalian cells, adhered on the polymer coated on the surface, wherein these animal and/or mammalian and/or stem cells are preferably not human embryonic stem cells.

Advantageously, the animal and/or mammalian cells are adhered or inoculated at a known (subconfluent) density.

Another related aspect of the present invention is the use of the composition of the present invention or of the polymer consisting essentially of the formula I for an in vitro culture of animal cells, preferably of mammalian cells, including stem cells (not human embryonic).

### Examples

### Example 1.

L929 fibroblasts have been seeded on TCPS (Tissue Culture polystyrene) or on PS multiwells (24 wells) adopting an initial cell density of 10 000 cells per cm². The cell culture has been realized in a DMEM culture medium enriched with Fetal Bovine Serum (FBS) (10 %) buffered at pH 7.4 (commercial DMEM solutions). The cell culture has been conducted at 37°C under 5 % CO₂ atmosphere according to the standard operation conditions typically used for animal cell culture. The evolution of cell density and morphology (spreading behavior, shape) over time have been observed with a reverse optical microscopy under visible non polarized light.

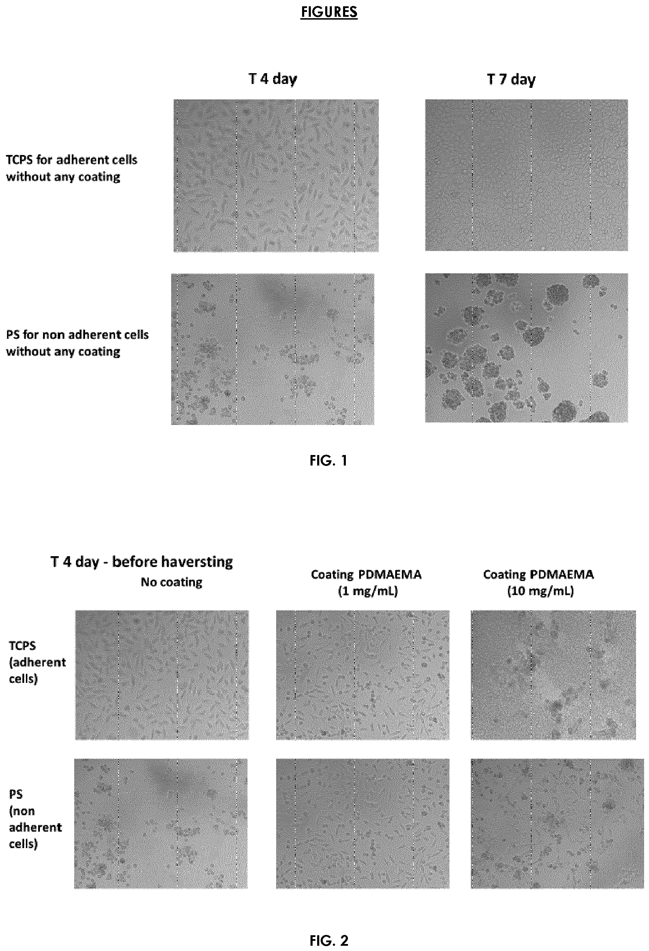

As reported on figure 1 the microscopic observations are highlighting that L929 cells cultured on TCPS are well spread after 4 days of culture while they appeared under the form of aggregates on non-functionalized PS. After 7 days of culture, fibroblasts are confluent and homogeneously distributed on TPCS, while they have generated big clusters on PS suitable for non-adherent cells.

### Example 2.

PDMAEMA (10 kDa) has been prepared by living radical polymerization. After synthesis this polymer has been purified by 4 purification steps in order to eliminate any monomer, catalyst and solvent residue.

PDMAEMA solutions of 1 and 10 mg/mL have been prepared by dissolving the freeze-dried polymer in ultra purified water. After dissolution the PDMAEMA solutions have been sterilized by filtration on 0.22 µm sterile filter within laminar flow.

PolyStyrene (TCPS) or non-modified polystyrene (PS) 24 Multiwell have been coated with the PDMAEMA solutions by physical impregnation. Briefly 0.5 mL of the PDMAEMA solutions have been incubated within the well for a duration of 30 min at room temperature under slight lateral agitation (500 rpm) under sterile conditions.

The solutions of PDMAEMA have been eliminated by aspiration and replaced by 0.5 mL of DMEM culture medium complemented with FBS (10%).

L929 fibroblasts have been seeded on each well by adding 0.5 mL of a L929 fibroblast cell suspension to achieve a cell density of 10.000 cells / cm2.

Cell culture and cell observation have realized as reported in example 1.

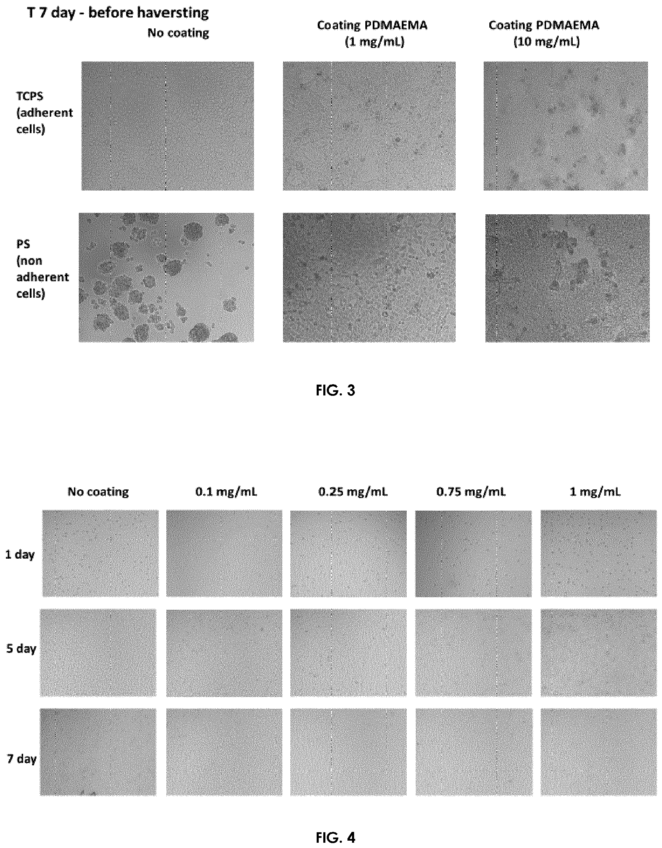

In contrast to the results outlined on example 1, the microscopic observations reported on figures 2 and 3 are demonstrating that the simple physical coating of PDMAEMA (1 mg/mL) on polystyrene foreseen for non-adherent animal cells (PS) allows the adhesion and proliferation of fibroblasts L929. Indeed, very few cell aggregates are noticed under microscopic observation and the cell density looks similar compared to the control made of TCPS without any coating for day 4 and 7.

Interestingly enough, the same PDMAEMA coating (1 mg/mL)) applied on polystyrene culture dishes made of TCPS does not interfere with the adhesion and proliferation of fibroblasts L929. Indeed, at day 4 the L929 cultivated on TCPS coated with PDMAMEA (1mg/mL) are well spread with the typical elongation morphology. But more heterogeneities in cell shape are however noticed compared to the observation done on the control TPCS. The same observations have been done with PS coated with PDMAEMA (1 mg/mL). The inventors have noticed less heterogeneities at lower PDMAEMA concentration, and almost no heterogeneities at about 0.03 mg/ml.

In contrast when both types of polystyrene wells are coated with a higher concentration of PDMAEMA solution (i.e. 10 mg/mL), this coating significantly impact the cell morphology for giving rise to cell aggregates already noticed at day 4 for both types of PS.

At day 7 the cell surface density are rather similar both for TCPS control, TCPS coated with PDMAMEA (1mg/mL) or PS coated with PDMAEMA (1 mg/mL). But as noticed on day 4, the homogeneity of L929 spreading and shape is better for control TCPS. Those observations are therefore in favor of a similar proliferation rate whatever the surface of PS.

### Example 3

Suspecting a toxicity effect of PDMAEMA coated on TCPS at the highest concentration used last time (i.e. 10 mg/mL), while at 1 mg/mL most of the cells were alive, the inventors have assessed lower PDMAEMA concentration to coat TCPS, using one of the following polymer concentration: 0.1 ; 0.25 and 0.75 and 1 mg/mL.

PDMAEMA solutions at a concentration of 0.1 ; 0.25 ; 0.75 or 1 mg/mL have been prepared by dissolving a PDMAEMA of a mean molecular weight of 10 kDa under a freeze-dried form in ultrapurified water. The PDMAEMA solutions have been sterilized by filtration on 0.22 µm sterile filter within laminar flow.

PolyStyrene (TCPS) 24 Multiwell have been coated with the PDMAEMA solutions by physical impregnation according to the experimental conditions already reported on example 2.

L929 fibroblasts have been seeded on each well by adding 0.5 mL of a L929 fibroblast cell suspension to achieve a cell density of 10.000 cells / cm².

Cell culture and cell observation have realized as reported in example 1.

As highlighted on figure 4, after 5 days of culture, the cell density is increasing when decreasing the concentration of PDMAEMA from 1 mg/mL to 0.1 mg/mL. When coated with a 0.1 mg/mL PDMAEMA solution no significant difference in cell density is noticed as compared to the control (without PDMAEMA) and in both cases cells are close to be confluent already.

At day 7 of culture, a higher density of cells is still observed at the lowest concentration of PDMAEMA, i.e. similar to the control, but the differences are lower. For the control and for PDMAEMA coating of 0.1 mg/mL, patches made from multilayers of cells can be visualized under microscopy.

### Example 4.

Fibroblasts cultured on Tissue Culture PolyStyrene (TCPS) or non-modified polystyrene (PS) as described on example 2 have been detached after 7 days of culture.

This cell harvesting has been performed, first by elimination of the culture medium followed by the addition of 0.2 mL of:
either a classical protease enzymatic solution made of trypsin and EDTA solution;
or a DMEM culture medium, made from a commercial DMEM solution but whose pH has been increased to 8.0 with a sodium hydroxide solution (1M).

Cell detachment has been realized in these medium for 5 minutes under slight lateral agitation at 500 rpm at 37°C under 5 % CO₂ atmosphere.

After this period, 0.5 mL of DMEM culture medium containing 10 % FBS have been added in each well.

Cells have been homogenized by 3 up-and-down aspirations with 1 mL pipette and transferred in Falcon tube of 15 mL before mixing under vortex stirrer, type ReaxTop, VWR, fixed at maximum speed n°6.

0.2 ml of each suspension have been withdrawn and transferred in 96 multiwell plates made of TCPS.

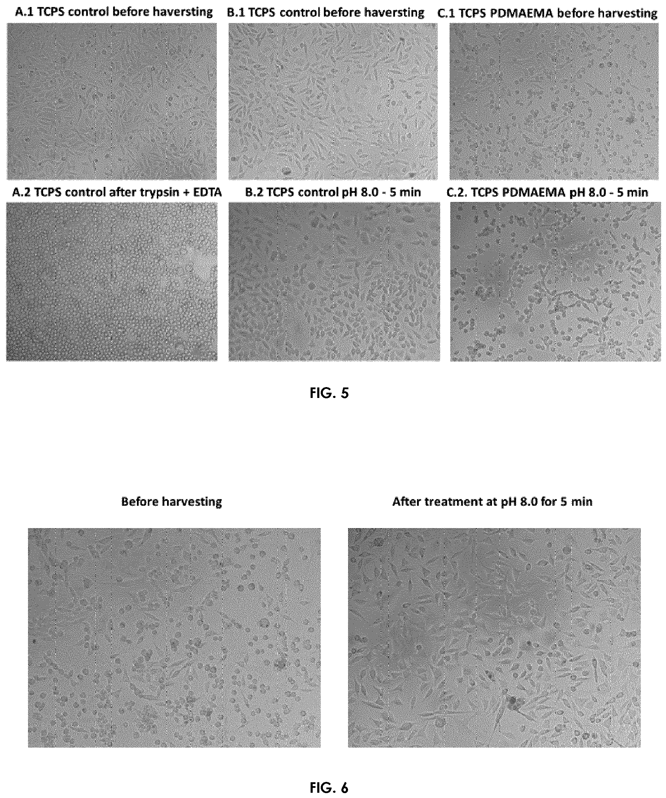

The detached cells have been observed under optical microscopy (figure 5).

In order to quantify cell detached from the multiwell and measure the viability of the cells, a MTT has been carried out according to the following way:
Cells have been incubated for 2h at 37°C under 5% CO₂ atmosphere to promote cell adhesion;
The supernatant of each well has been removed carefully by aspiration;
Cell viability and cell counting have been realized using the MTT assay. Briefly,
   - 0.2 mL of MTT reagent (0.5 mg MTT /ml of DMEM without FBS)
   - Incubation for 2h at 37°C under 5 % CO₂ atmosphere
   - Removing of the supernatant carefully by aspiration
   - Addition of 0.2 mL of DMSO
   - Incubation of the multiwell for 2h at room temperature under slight lateral agitation (500 rpm)
   - The optical densities (DO) of the wells being part of the multiwell are measured at 540 nm using a commercial multiwell reader.
   - The DOs are converted in terms of cell number per well adopting a calibration curve made using multiwells containing well-known amount of cells per well, i.e. ranging between 5,000 to 80,000 and incubated in the same conditions as reported above for the experimental assays.

After trypsinisation in the presence of EDTA, L929 cells are released from the TCPS surface (figures 5 A.1 and 5 A.2). After being released from the TCPS surface, the fibroblasts have a spherical shape.

The alkalinisation of the culture medium to pH 8.0 for 5 min does not affect cell adhesion cultured on native TCPS (figures 5 B.1 and 5 B.2). Indeed, cells remain mostly attached and spread on TCPS coated with PDMAEMA after the alkalinisation treatment.

In contrast, if TCPS has been previously coated with PDMAEMA (1 mg/mL), the slight alkalinisation of DMEM to pH 8.0 for 5 min allows a significant harvesting of fibroblasts L929 which disclose also a spherical shape (figures 5 C.1 and 5 C.2).

On the other hand, microscopic observations given on figure 6 are indicating that L929 cultivated on PS coated with PDMAEMA (1 mg/mL) have remained mostly attached to this substrate after the slight alkalinisation of DMEM to pH 8.0 for 5 min.

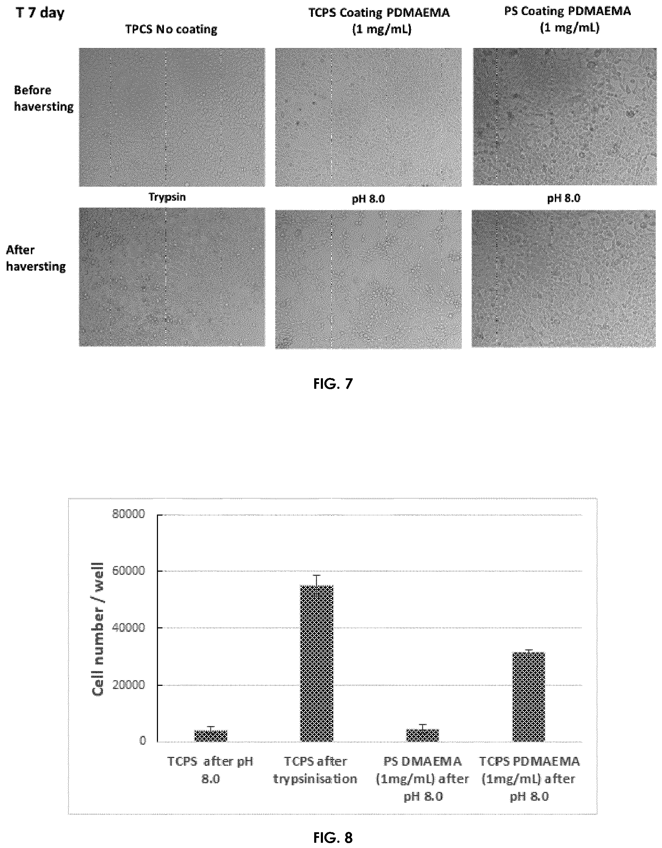

Similar microscopic observations of fibroblasts behavior before and after harvesting on either non coated or PDMAEMA coated TCPS or PS have been also noticed after 7 days of culture as disclosed on figure 7.

The MTT assays has confirmed the qualitative observations obtained by microscopy giving access to the total amount of cells per surface unit. Moreover, it should be reminded that this test counts only viable cells. The results outline on figure 8 the viability of L929 after their cultivation on TCPS coated PDMAEMA (1 mg/mL) and their detachment by incubation at pH 8.0 for 5 min. The total amount of fibroblasts recovered per well is however lower (57 %) compared to the TCPS control, i.e. without any PDMAEMA coating and after trypsinisation.

This difference could result from a difference in proliferation rate of the cells, or/and to a difference in harvesting efficiency between these two experimental conditions.

The MTT results allows to notice that the cell cultured on PS coated with PDMAEMA are not efficiently detached after the slight alkalinisation of DMEM to pH 8.0 in contrast to what has been observed with TCPS coated with the same polymer. Accordingly, the combination of the results coming from microscopic observations and of MTT counting allows to conclude that this lower number of viable cells recovered from PS surface coated with PDMAEMA (1mg/mL) is not arising from their low proliferation rate on this surface, but from their poor detachment PS surface coated with PDMAEMA (1 mg/mL).

This surprising difference in harvesting efficiency of L929 cultured on either TCPS or PS, both physically coated with the same polymer in the same experimental conditions is explained by the inventors by the fact that TCPS is bearing anionic groups (sulfonate), while PS foreseen for the culture of non-adherent cells should be hydrophobic and neutral. According to these differences in surface chemistry between TCPS and PS, the inventors consider a difference in adsorption behavior between PDMAEMA and these two polymeric surfaces. In the case of TCPS the inventors expect that ionic interactions prevail, while polar or van der Waals interactions forces mostly promote the anchorage of PDMAEMA on non PS.

Accordingly, the inventors have shown that a slight increase of the pH of DMEM culture medium to 8.0, i.e. sufficient to mostly neutralize the ternary amine of PDMAEMA, is enough to promote its release from the surface of TCPS with the detachment of cells, which have adhered originally thanks to the cationic group of this polycation physically coated on the TCPS surface.

In the case of non-functionalized PS, the inventors anticipate that polar forces between PDMAEMA and PS should not be influenced by the pH, therefore explaining why fibroblasts have remained attached on PS after the slight alkalinisation to pH 8.0.

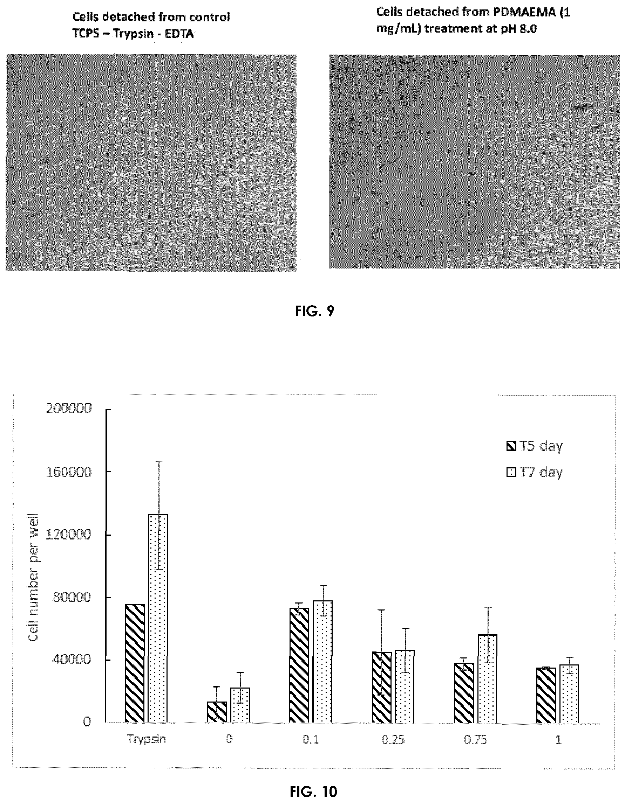

In addition to the demonstration of the viability of L929 after their culture on TCPS coated on PDMAEMA (1mg/mL) and their harvesting by a slight alkalinisation at pH 8.0 for 5 min, the inventors have also verified their ability to adhere and spread quickly after their seeding on TCPS. As disclosed on figure 9, no significant difference in cell phenotype behavior between fibroblasts either detached by trypsin from control TCPS or either detached from PDMAEMA coated TCPS.

### Example 5.

L929 harvesting after their culture 7 days on TCPS after their coating with PDMAEMA at a concentration of 0 ; 0.1 ; 0.25 ; 0.75 or 1 mg/mL.

After cell adhesion and proliferation on these modified surfaces, the inventors have compared the cell amount recovered after trypsin treatment or alkalinization and have noted that a slight alkalinisation of the medium (up to maximum pH 8.5) detach the cells without any trypsinisation and treatment with EDTA.

Fibroblasts cultured on Tissue Culture PolyStyrene (TCPS) coated with PDMAEMA at a concentration of 0 ; 0.1 ; 0.25 ; 0.75 or 1 mg/mL as described on example 4 have been detached after 5 or 7 days of culture.

This cell harvesting has been performed, first by elimination of the culture medium followed by the addition of a trypsin solution or an incubation at pH 8.0.

The results given on figure 10 is highlighting that:
- At day 5 day of culture:
   No significant cell detachment is noticed for TCPS without PDMAEMA coating after incubation at pH 8.0.

A similar cell detachment efficiency and cell viability is noticed between the trypsin control and the TCPS wells coated with the lowest concentration of PDMAEMA (0.1 mg/mL).

Above this polymer concentration, the amount of cells recovered after the slight alkalinisation is decreasing, thus in agreement with the difference in cell density noticed under microscopical observations outlined on figure 4.
- At day 7 day of culture:
   A similar amount of cells are recovered from wells coated with PDMAEMA compared to day 5, i.e. with a decrease in cell number with the concentration of PDMAEMA solution used to perform the coating.

The cell recovery for the trypsin control is 60 % higher, as compared to the wells coated with the PDMAEMA: the higher cell density reduces the effect of the alkalinization.

To sum up, the inventors consider that the adhesion, proliferation rate, shape, viability and detachment efficiency of fibroblasts cultivated on PDMAEMA coated TCPS are similar compared to control TCPS if concentration of the PDMAEMA solution used to coat the substrate is below 0.2 mg/mL and if cells are not cultivated above confluency.

### Example 6.

PDMAEMA solutions of 1 mg/mL have been prepared by dissolving the freeze-dried polymer in ultrapurified water as outlined on example 2. After dissolution the PDMAEMA solutions have been sterilized by filtration on 0.22 µm sterile filter within laminar flow.

PolyStyrene (TCPS) 24 Multiwells have been coated with the PDMAEMA solutions by physical impregnation. Briefly 0.5 mL of the PDMAEMA solutions have been incubated within the well for a duration of 30 min at room temperature under slight lateral agitation (500 rpm) under sterile conditions.

The solutions of PDMAEMA have been eliminated by aspiration and the coated 24 Multiwells plate has been dried in a vacuum oven at 40°C overnight.

The PDMAEMA-coated 24 Multiwells has been re-coated with the PDMAEMA solutions by physical impregnation by repeating the same coating procedure as described above in order to have a double PDMAEMA coating, i.e. made from two polymer coatings. Briefly, 0.5 mL of the PDMAEMA solutions have been incubated within the PDMAEMA coated 24 Multiwells for a duration of 30 min at room temperature under slight lateral agitation (500 rpm) and under sterile conditions.

The solutions of PDMAEMA have been eliminated by aspiration and replaced by 0.5 mL of DMEM culture medium complemented with FBS (10%).

L929 fibroblasts have been seeded on each well by adding 0.5 mL of a L929 fibroblast cell suspension to achieve a cell density of 10.000 cells / cm2.

Cell culture and cell observation have realized as reported in example 1.

### Example 7.

Vero cells are known to recover very slowly after freezing. Therefore, several weeks are known to be necessary to recover them before proceeding to their first passage on a new TCPS surface. In order to enhance their *in vitro* recovery, adhesion, spreading and proliferation, we have seeded them after thawing on TCPS first coated with PDMAEMA.

As outlined on example 2 PolyStyrene (TCPS) 24 Multiwells have been coated with the PDMAEMA solutions by physical impregnation. Briefly 0.5 mL of the PDMAEMA solutions has been incubated within the well for a duration of 30 min at room temperature under slight lateral agitation (500 rpm) under sterile conditions.

The solutions of PDMAEMA have been eliminated by aspiration and replaced by 0.5 mL of DMEM culture medium complemented with FBS (10%).

1 mL of Vero cell suspension, i.e. cell line isolated from kidney epithelial cells extracted from an African green monkey, stored in liquid nitrogen, has been quickly thawed at 37°C and diluted afterwards in 9 mL of DMEM containing 15% FBS. This cell suspension has been centrifuged for 5 min at 200 g in order to eliminate DMSO. The supernatant has been discarded by aspiration and the cell pellet has been suspended in 3 mL of DMEM containing 15% FBS by vortexing for 10 sec. 1.5 mL of this cell suspension has been transferred in one well of PolyStyrene (TCPS) 12 Multiwells coated with PDMAEMA. As a control, 1.5 mL of the same cell suspension has been transferred in one well of PolyStyrene (TCPS) 12 Multiwells without PDMAEMA coating.

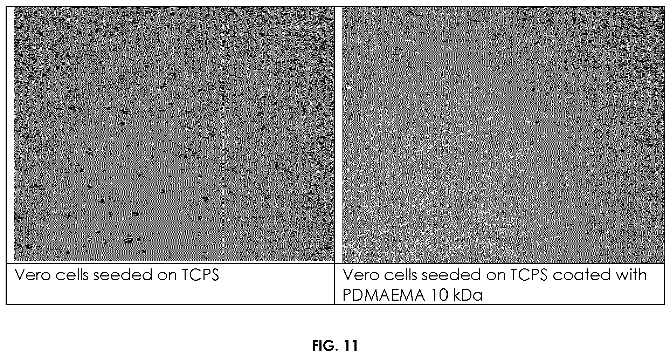

Cell culture and cell observation have been realized as reported in example 1. As highlighted on figure 11, Vero cells seeded on PDMAEMA-coated TCPS have quickly adhered, spread and proliferated on this surface after 1 week. In contrast the same cells cultured on non-coated TCPS have not adhered at all, disclosing spherical shape after the same period of incubation.

It should be understood that the present invention is not limited to the described embodiments and that variations can be applied without going outside of the scope of the claims.

## Claims

1. A method to grow animal cells, preferably mammalian cells and/or stem cells, comprising the steps of:
- selecting a surface,
- coating the said surface with a polymer substantially consisting of a succession of monomers according to the formula I 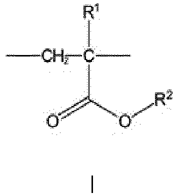 and
- growing the said animal (mammalian and/or stem) cells on the said coated surface for a period of time so that the said animal (mammalian and/or stem) cells adhere to the said coated surface, wherein R¹ represents a hydrogen atom or a straight or branched chain alkyl group from 1 to 6 carbon atoms;
wherein R² represents a straight or branched chain alkyl group from 1 to 6 carbon atoms, which is substituted by a protonable amino group;
wherein the said polymer has a mean pKa between 6.0 and less than 7.5 and a charge density at pH 7.0 between 20 and 50% (number of the positively charged monomers:total number of monomers), wherein the said animal and/or mammalian and/or stem cells are not human embryonic stem cells.

2. The method according to claim 1, wherein the surface is negatively charged.

3. The method of claim 1 or 2, further comprising the step of detaching the grown animal cells upon increasing the pH of the medium to a pH comprised between 7.8 and 8.2.

4. The method according to any one of the preceding claims, wherein the polymer is comprising more than 80%, preferably more than 90% and more preferably more than 95% of the monomers according to the formula I, such as between 95% and 99% (number of the monomers according to the formula I:total number of the monomers), and/or wherein R¹ is a methyl, and/or wherein the polymer harbors a charge density at pH 7,0 between 25 and 40%, preferably between 30 and 35% (number of the positively charged monomers:total number of monomers), and/or harbors less than 10% of negative charge density at pH 7,0, preferably less than 5%, more preferably less than 2% (number of the negatively charged monomers:total number of monomers).

5. The method according to any one of the preceding claims, wherein the polymer is coated on the surface upon incubation at a concentration between 0.01 mg/ml and 1.0 mg/ml, preferably 0.05 mg/ml and 0.5 mg/ml, more preferably between 0.1 mg/ml and 0.3 mg/ml.

6. The method according to any one of the preceding claims, wherein the polymer is according to the formula II 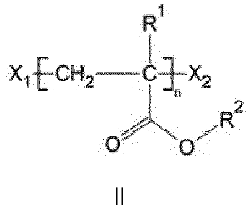
wherein X₁ and X₂ represent the alpha and omega end groups of the said polymer;
wherein the said X₁ and X₂ independently represent a hydrogen atom, a hydroxyl group, an ethyl isobutyrate group, an alkyl group, a halogen group, a carboxylic acid group, an amino group, a methoxy group or an ethoxy group and wherein n is the number of the repetitive monomer units according to the formula I.

7. A composition comprising a surface coated with a polymer substantially consisting of a succession of monomers according to the formula I 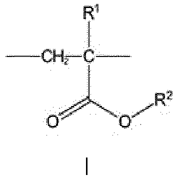
wherein R¹ represents a hydrogen atom or a straight or branched chain alkyl group from 1 to 6 carbon atoms;
wherein R² represents a straight or branched chain alkyl group from 1 to 6 carbon atoms, which is substituted by a protonable amino group; preferably, wherein the said polymer has a mean pKa between 6.0 and 7.5 and/or a charge density at pH 7.0 between 20 and 50% (number of the positively charged monomers:total number of monomers).

8. The composition of claim 7, wherein the surface is negatively charged, preferably wherein the surface is selected from the group of plasma-treated polystyrene, polyelectrolytes or polyelectrolytes complexes or polyampholytes, biopolymer, inorganic particles(hydroxyapatite), glass.

9. The composition according to any one of the preceding claims 7 to 8, wherein the polymer is comprising more than 80%, preferably more than 90% and more preferably more than 95% of the monomers according to the formula I, such as between 95% and 99% (number of the monomers according to the formula I:total number of the monomers), and/or wherein R¹ is a methyl group.

10. The composition according to any one of the preceding claims 7 to 9, wherein the polymer harbors a charge density at pH 7,0 between 25 and 40%, preferably between 30 and 35% (number of the positively charged monomers:total number of monomers), and/or harbors less than 10% of negative charge density at pH 7,0, preferably less than 5%, more preferably less than 2% (number of the negatively charged monomers:total number of monomers).

11. The composition according to any one of the preceding claims 7 to 10, wherein the polymer is coated on the surface upon incubation at a concentration between 0.01 mg/ml and 1.0 mg/ml, preferably 0.05 mg/ml and 0.5 mg/ml, more preferably between 0.1 mg/ml and 0.3 mg/ml.

12. The composition according to any one of the preceding claims 7 to 11 further comprising animal cells, preferably mammalian cells and/or stem cells adhered on the polymer, wherein the said animal and/or mammalian and/or stem cells are not human embryonic stem cells.

13. The composition of claim 12, wherein the animal and/or mammalian and/or stem cells are adhered or inoculated at a known density.

14. Use of the composition according to any one of the preceding claims 7 to 13 or of a polymer substantially consisting of a succession of monomers according to the formula I 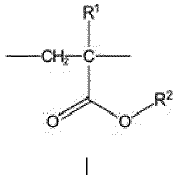
wherein R¹ represents a hydrogen atom or a straight or branched chain alkyl group from 1 to 6 carbon atoms;
wherein R² represents a straight or branched chain alkyl group from 1 to 6 carbon atoms, which is substituted by a protonable amino group;
preferably, wherein the said polymer has a mean pKa between 6.0 and 7.5 and/or a charge density at pH 7.0 between 20 and 50% (number of the positively charged monomers:total number of monomers)
for an *in vitro* culture of animal cells, preferably of mammalian cells and/or of stem cells.

15. A pharmaceutical composition comprising the animal (mammalian and/or stem) cells or parts thereof or the secretion products thereof, obtainable by the method according to any one of the preceding claims 1 to 6.
